# FASCICULE DE BREVET EUROPEEN

(11) **EP 1 373 176 B1**
(45) Date de publication et mention de la délivrance du brevet: **16.11.2016**
(21) Numéro de dépôt: 02724395.5
(22) Date de dépôt: 02.04.2002
(51) Int. Cl.: C07C 35/44, C07C 29/76

(54) **EXTRAIT DE COQUES DE GRAINES DE LUPIN CONTENANT DU LUPEOL**
LUPEOL ENTHALTENDER AUSZUG AUS LUPINKÖRNERSCHALEN
EXTRACT FROM THE PODS OF LUPIN SEEDS CONTAINING LUPEOL

(30) Priorité: 03.04.2001 FR 0104493
(43) Date de publication de la demande: 02.01.2004
(73) Titulaire: Laboratoires Expanscience, 92419 Courbevoie Cedex (FR)
(72) Inventeur: PICCIRILLI, Antoine, F-78000 Versailles (FR); LEGRAND, Jacques, F-61290 Neuilly sur Eure (FR); BROUTIN, Nicole, F-28800 Alluyes (FR)
(74) Mandataire: Regimbeau
(86) Numéro de dépôt international: PCT/FR2002/001130
(87) Numéro de publication internationale: WO 2002/085827

(56) Documents cités:
- WO-A-95/35103
- FR-A- 2 762 512
- J.E. GEARIEN, ET AL.: "Structure of lupeol and its 19alpha-H-isomer" JOURNAL OF PHARMACEUTICAL SCIENCES, vol. 64, no. 1, janvier 1975 (1975-01), pages 152-154, XP002193339 American Pharmaceutical Association, Washington, DC, US ISSN: 0022-3549
- B.J.F. HUDSON, ET AL.: "Oil content, fatty acids and unsaponifiable lipids of lupin seed" JOURNAL OF PLANT FOODS, vol. 5, no. 1, 1983, pages 15-21, XP001064436 London, GB
- H. DIETERLE: "Über Lupeol", ARCHIV DER PHARMAZIE, vol. 261, no. 2, 1923, pages 89-98, VERLAG CHEMIE. WEINHEIM. ISSN: 0365-6233

## Description

La présente invention concerne un procédé d'obtention d'un extrait de coques de graines de lupin contenant du lupéol, en particulier d'un extrait riche en lupéol.

Le lupin est une plante assez répandue, que l'on trouve en Europe, en Asie, ainsi qu'en Amérique du Nord et du Sud. Ce végétal est un proche parent du pois, de la fève, du soja et du haricot. Plusieurs espèces de lupin peuvent être citées comme étant les plus connues : lupinus albus (lupin blanc), lupinus angustifolius (lupin bleu), lupinus luteus (lupin jaune), lupinus mutabilis (lupin changeant), lupinus graecus, lupinus micranthus Guss, lupinus hispanicus, lupinus pilosus, lupinus cosentinii, lupinus atlanticus, lupinus princei et lupinus somaliensis. Une des espèces les plus courantes sur le territoire européen est le lupin blanc doux (lupinus albus), notamment la variété Arès présentant le gène pauper.

Alors que les graines de lupin sont traditionnellement employées en tant qu'engrais ainsi que dans l'alimentation humaine et animale pour leur forte teneur en protéines, les coques ou pellicules de graines de lupin n'ont que très rarement été utilisées dans l'industrie. Elles constituent néanmoins une source naturelle riche en lupéol.

Le lupéol (1) appartient à la famille des triterpènes et plus particulièrement à celle des alcools triterpéniques.

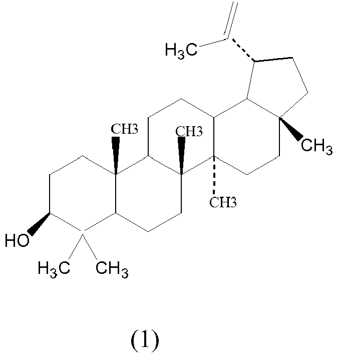

Le lupéol présente un intérêt certain de par ses nombreuses activités biologiques. Il est notamment connu pour ses propriétés anti-inflammatoires (Singh S. et coll., Filoterapia, 1997, 68, No. 1, 9) et analgésiques (De Miranda A. L. et coll., Planta Med, 2000, 66(3), 284), son action nephroprotectrice vis-à-vis des métaux lourds (Nagaraj M. et coll., J. Appl. Toxicol, 2000, 20(5), 413), son action antihistaminique (De Medrano Villar M. J. et coll., Methods Find Exp. Clin. Pharmacol., 1997, 19, No. 8, 515), ses activités anti-mitotiques (Zachariah R. et coll., Indian J. Pharm. Sci., 1994, 56, No. 4, 129) et anti-virales (Kahlos K., Filoterapia, 1996, 67, No. 4, 344).

Le lupéol peut également être utilisé en tant qu'intermédiaire de synthèse, notamment pour la préparation de phyto-hormones et d'analogues de stéroides.

Le lupéol est présent dans de nombreux végétaux tels que l'Aloe vera ou l'écorce de Crataeva nurvala. Il a été isolé à plusieurs reprises à partir de plantes diverses comme le Bresk. Il n'a cependant jamais été extrait des coques de lupin. Une huile de lupin contenant du lupéol a été extraite à partir de graines de lupin dans le brevet FR 2 762 512, mais l'huile provenait alors des graines préalablement décortiquées et donc débarrassées de leurs coques, et le lupéol contenu dans l'huile présentait une teneur maximale de 0,5% en poids par rapport à la composition totale de l'huile.

Or, les coques de lupin constituant une source potentielle riche en lupéol, de bon marché et de bien meilleure disponibilité que la plupart de ses homologues, il existait ainsi un besoin d'isoler un extrait de coques de graines de lupin, notamment riche en lupéol, et de mettre au point un procédé d'obtention d'un tel extrait. Le lupin en général, et le lupin blanc (lupinus albus) en particulier, est en effet un oléoprotéagineux cultivé à grande échelle, avec les techniques agricoles modernes, par opposition aux plantes moins répandues qui contiennent du lupéol, à l'instar de l'Aloe vera et de Crataeva nurvala.

Le document « Über Lupeol » de H. Dieterle (Archiv ver Pharmazie, 1923, 261(2), 89-98) semble divulguer un procédé d'extraction du lupéol par extraction des coques de lupin avec de l'éther. Il est difficile d'identifier le lupéol dans ce document en raison de structures chimiques non cohérentes. En outre les étapes du procédé sont différentes de celles du procédé selon l'invention.

La publication scientifique de J.E. GEARIEN et al. (Journal of Pharmaceutical Sciences, Vol. 64, no. 1, January 1975, 152-154) porte sur la détermination de la structure du lupéol et de ses isomères.

La demande internationale WO 95/35103 porte sur une composition pharmaceutique comprenant du *β*-lupeol en tant qu'agent antiviral.

La publication scientifique de B. J. F. HUDSON et al. (Journal of Plant Foods (1983) 5, 15-21) porte sur l'étude de l'utilisation possible de quatre espèces de *Lupinus* en tant que source d'huile comestible. Pour extraire l'huile, les graines de lupin entières sont broyées puis extraites dans l'hexane.

La présente invention a ainsi pour objet un procédé d'obtention d'un extrait de coques de graines de lupin contenant du lupéol. Avantageusement, ledit extrait présente un taux de lupéol supérieur à 30% en poids, de préférence supérieur à 50% en poids. De manière encore plus avantageuse, ledit extrait de coques de graines de lupin présente un taux de lupéol compris entre 70 et 100%.

Par le terme de "coque de graine de lupin", on entend au sens de la présente invention, la pellicule entourant la graine. Les coques représentent en moyenne 15% en poids de la graine sèche.

L'extrait obtenu par le procédé selon la présente invention est préparé à partir de coques de graines de lupin issues de l'opération de décorticage mécanique des graines.

Dans un mode de réalisation particulier de la présente invention, le lupin est choisi dans le groupe constitué par le lupinus angustifolius, le lupinus albus, le lupinus luteus, le lupinus mutabilis, le lupinus graecus, le lupinus micranthus Guss, le lupinus hispanicus, le lupinus pilosus, le lupinus cosentinii, le lupinus atlanticus, le lupinus princei et le lupinus somaliensis. Selon la présente invention, le lupin est avantageusement le lupinus albus (lupin blanc doux), de genre européen, de préférence la variété Arès portant le gène pauper.

Le procédé selon la présente invention comprend au moins la succession d'étapes suivantes :
- broyage des coques de lupin,
- extraction des lipides totaux contenus dans les coques de lupin broyées à l'aide d'un solvant organique choisi dans le groupe constitué par les alcanes aliphatiques, les alcanes aromatiques, les alcools aliphatiques et leurs dérivés halogénés, et
- purification des lipides obtenus pour obtenir un extrait riche en lupéol.

Les coques isolées des graines sont broyées à l'aide d'un broyeur à cylindre ou à marteaux. Le procédé d'obtention d'un extrait riche en lupéol selon la présente invention repose sur l'extraction des lipides contenus dans les coques de lupin à l'aide d'un solvant organique. Avantageusement, le solvant organique est l'hexane. Les coques épurées présentent généralement une teneur en lipides totaux comprise entre 0,5 et 5% en poids par rapport au poids total des coques.

Par le terme d'extrait "riche en lupéol", on entend au sens de la présente invention, un extrait présentant un taux de lupéol supérieur à 30% en poids, avantageusement supérieur à 50% en poids, et de manière encore plus avantageuse compris entre 70 et 100% en poids.

La purification des lipides obtenus pour obtenir un extrait riche en lupéol selon la présente invention est mise en oeuvre alternativement par les procédés B et C décrits ci-dessous.

Dans un premier mode de réalisation de la présente invention (procédé B), la purification des lipides totaux extraits des coques de lupin comprend au moins la succession d'étapes suivantes :
- évaporation du solvant sous vide, puis dissolution dans un alcool aliphatique,
- saponification,
- cristallisation par refroidissement, et
- filtration et/ou lavage et/ou essorage.

Selon la présente invention, l'alcool aliphatique est avantageusement l'éthanol et le solvant utilisé lors de l'étape de saponification est avantageusement la potasse alcoolique. Après saponification, la solution hydro-alcoolique obtenue est soumise à une cristallisation fractionnée par refroidissement. Après filtration et/ou lavage et/ou essorage, l'extrait solide obtenu peut présenter une teneur en lupéol d'au moins 30 % en poids, avantageusement d'au moins 50 % en poids, de manière encore plus avantageuse d'au moins 60 % en poids. L'extrait obtenu riche en lupéol peut ensuite être purifié par une ou plusieurs étape(s) de recristallisation, notamment dans l'hexane.

Dans un second mode de réalisation de la présente invention (procédé C), la purification des lipides totaux extraits des coques de lupin comprend au moins la succession d'étapes suivantes :
- évaporation du solvant sous vide, puis dissolution dans un alcool aliphatique,
- saponification,
- extraction liquide-liquide des insaponifiables des lipides de coques de lupin à l'aide d'un solvant organique choisi dans le groupe constitué par les alcanes aliphatiques et leurs dérivés halogénés, et
- purification des insaponifiables par lavage, de préférence à l'eau, puis évaporation des solvants sous vide et séchage.

Selon la présente invention, il est ainsi possible d'obtenir un extrait riche en lupéol en procédant à une saponification du milieu réactionnel contenant les lipides totaux extraits des coques de lupin, puis à une extraction de la fraction insaponifiable des lipides de coques de lupin.

Par le terme d'"insaponifiable", on entend au sens de la présente invention, la fraction d'un corps gras qui, après action prolongée d'une base alcaline, reste insoluble dans l'eau et peut être extraite par un solvant organique.

Selon la présente invention, l'extraction liquide-liquide est mise en oeuvre dans une colonne pulsée à contre-courant en présence d'un solvant présentant une grande affinité vis-à-vis des insaponifiables, tel que les alcanes aliphatiques et leurs dérivés halogénés. Avantageusement selon la présente invention, le solvant organique pour l'étape d'extraction liquide-liquide est le dichloroéthane. La phase organique en sortie de colonne est alors lavée à l'eau, puis évaporée sous vide et le résidu est séché. L'extrait obtenu riche en lupéol peut ensuite être purifié par une ou plusieurs étape(s) de recristallisation, notamment dans l'hexane.

Toutes les étapes de procédés selon la présente invention sont bien connues de l'homme du métier.

Les exemples suivants sont donnés à titre non limitatif et illustrent la présente invention.

### Exemples de réalisation de l'invention

La matière première utilisée est constituée des coques de graines de lupin blanc doux de la variété Arès, portant le gène pauper. La teneur en lipides totaux des coques de lupin est égale à 1,8% en poids.

*1. Extraction des lipides totaux :* 75 kg de coques de lupin sont préalablement broyés à l'aide d'un broyeur à marteaux. Le broyat obtenu est ensuite chargé dans un filtre agité de type GUEDU afin de subir 8 lavages successifs à l'hexane (90 litres d'hexane engagés par lavage). La température d'extraction est fixée à 45°C. Chaque lavage comprend : un envoi de solvant frais dans le filtre, une agitation de la suspension pendant 15 minutes, et enfin une filtration de 15 minutes afin de récupérer le miscella. En fin de processus, les coques de lupin délipidées sont soufflées à la vapeur d'eau, sous vide, à une température de 50°C, afin d'extraire du gâteau le miscella résiduel. Les phases miscella sont enfin rassemblées, puis évaporées sous vide, par injection de vapeur sous vide, à 120°C. Cette opération est contrôlée de façon à obtenir une solution hexanique A présentant une teneur en lipides totaux comprise entre 15 et 30 %, préférentiellement 20% en poids.

### 2. Extraction du lupéol :

### 2.1 Procédé A (comparatif)

La solution hexanique A, obtenue lors de l'extraction des lipides totaux, est refroidie à 20°C et laissée sous agitation lente pendant 12 heures. Elle est ensuite filtrée sur büchner de façon à récupérer l'extrait cristallisé riche en lupéol. Cet extrait est enfin lavé par de l'hexane froid (15°C) puis séché à 60°C dans une étuve à vide pendant 48 heures.

L'extrait A obtenu se présente sous la forme d'une poudre jaune clair, titrant 76 % en lupéol. Le rendement global d'extraction du lupéol est de 72 %.

### 2.2 Procécé A suivi d'une étape de recristallisation (comparatif)

500 g de l'extrait A obtenu à l'exemple 1 sont purifiés par recristallisation dans l'hexane. L'extrait solide est en effet dissout dans l'hexane (solution à 10% en poids). Le mélange est porté à 70 °C à reflux pendant 15 minutes, jusqu'à solubilisation totale de l'extrait. La température est alors ramenée à 20°C et maintenue sous agitation lente jusqu'à précipitation totale du lupéol. Ce dernier est ensuite filtré, lavé par de l'hexane froid, puis enfin séché à 60°C dans une étuve à vide pendant 48 heures.

L'extrait B obtenu se présente sous la forme d'une poudre blanche, titrant 95% en lupéol. Le rendement global d'extraction du lupéol est de 65 %.

### 2.3 Procédé B :

2 kg de solution hexanique A obtenue lors de l'extraction des lipides totaux sont totalement évaporés, sous un vide de 200 mbar et par injection de vapeur vive à 120°C. Le solide obtenu est alors repris par 900 ml d'éthanol. Cette nouvelle solution éthanolique est alors saponifiée dans un réacteur agité, muni d'une double enveloppe, en présence de 240 g de potasse alcoolique à 50%. La réaction de saponification est effectuée à reflux pendant 4 heures et à une température de 70°C. La solution savonneuse est ensuite ramenée à une température de 20°C et maintenue sous agitation lente pendant 24 heures jusqu'à précipitation totale du lupéol. Ce dernier est ensuite filtré, lavé par de l'hexane froid, puis enfin séché à 60°C dans une étuve à vide pendant 48 heures.

L'extrait solide obtenu se présente sous la forme d'une poudre blanchâtre, titrant 84 % en lupéol. Le rendement global d'extraction du lupéol est de 91 %.

### 2.4 Procédé C :

2 kg de solution hexanique A obtenue lors de l'extraction des lipides totaux sont totalement évaporés, sous un vide de 200 mbar, par injection de vapeur vive à 120°C. Le solide obtenu est alors repris par 900 ml d'éthanol. Cette nouvelle solution éthanolique est alors saponifiée dans un réacteur agité, muni d'une double enveloppe, en présence de 240 g de potasse alcoolique à 50%. La réaction de saponification est effectuée à reflux pendant 4 heures et à une température de 70°C. La solution savonneuse obtenue est ensuite diluée par de l'eau adoucie (dilution 50/50 volumique). Cette nouvelle solution hydro-alcoolique est alors envoyée au pied d'une colonne pulsée à contre courant. La tête de la colonne est alimentée quant à elle en 1,2-dichloroéthane utilisé comme solvant d'extraction du lupéol. La phase organique obtenue subit alors les opérations unitaires classiques de purification suivantes :
1) lavage à l'eau dans une colonne à contre-courant
2) évaporation sous vide dans un évaporateur rotatif
3) séchage du résidu dans une étuve à vide (70°C, 48 heures).

L'extrait C obtenu se présente sous la forme d'une poudre jaune clair, titrant 85 % en lupéol. Le rendement global d'extraction du lupéol est de 87 %.

### 2.5 Procécé C suivi d'une étape de recristallisation :

200 g d'extrait C obtenu à l'exemple 3 peuvent être purifiés par recristallisation dans l'hexane. L'extrait solide est en effet dissout dans l'hexane (solution à 10%). Le mélange est porté à 70 °C à reflux pendant 15 minutes, jusqu'à solubilisation totale de l'extrait. La température est alors ramenée à 20°C et maintenue sous agitation lente jusqu'à précipitation totale du lupéol. Ce dernier est ensuite filtré, lavé par de l'hexane froid puis enfin séché à 60°C dans une étuve à vide pendant 48 heures.

L'extrait D obtenu se présente sous la forme d'une poudre blanche, titrant 96,5 % en lupéol. Le rendement global d'extraction du lupéol est de 70 %.

## Revendications

1. Procédé d'obtention d'un extrait de coques de graines de lupin contenant du lupéol, **caractérisé en ce qu'**il comprend au moins la succession d'étapes suivantes :
- broyage des coques de lupin,
- extraction des lipides totaux contenus dans les coques de lupin broyées à l'aide d'un solvant organique choisi dans le groupe constitué par les alcanes aliphatiques, les alcanes aromatiques, et leurs dérivés halogénés, et
- purification des lipides obtenus pour obtenir un extrait riche en lupéol, **caractérisé en ce que** la purification des lipides obtenus comprend au moins la succession d'étapes suivantes :
- évaporation du solvant sous vide, puis dissolution dans un alcool aliphatique,
- saponification,
- cristallisation par refroidissement, et
- filtration et/ou lavage et/ou essorage.

2. Procédé d'obtention d'un extrait de coques de graines de lupin contenant du lupéol, **caractérisé en ce qu'**il comprend au moins la succession d'étapes suivantes :
- broyage des coques de lupin,
- extraction des lipides totaux contenus dans les coques de lupin broyées à l'aide d'un solvant organique choisi dans le groupe constitué par les alcanes aliphatiques, les alcanes aromatiques, et leurs dérivés halogénés, et
- purification des lipides obtenus pour obtenir un extrait riche en lupéol, **caractérisé en ce que** la purification des lipides obtenus comprend au moins la succession d'étapes suivantes :
- évaporation du solvant sous vide, puis dissolution dans un alcool aliphatique,
- saponification,
- extraction liquide-liquide des insaponifiables de coques de lupin à l'aide d'un solvant organique choisi dans le groupe constitué par les alcanes aliphatiques et leurs dérivés halogénés, et
- purification des insaponifiables par lavage, puis évaporation des solvants sous vide et séchage.

3. Procédé selon la revendication 1 ou 2, **caractérisé en ce que** le solvant organique est l'hexane.

4. Procédé selon la revendication 2 ou 3, **caractérisé en ce que** le solvant organique pour l'étape d'extraction liquide-liquide est le dichloroéthane.

5. Procédé selon l'une quelconque des revendications 1 à 4, **caractérisé en ce qu'**il comprend en outre une ou plusieurs étape(s) finale(s) de recristallisation.

6. Procédé selon l'une quelconque des revendications précédentes, **caractérisé en ce que** l'extrait obtenu présente un taux de lupéol supérieur à 30% en poids, de préférence supérieur à 50% en poids.

## Patentansprüche

1. Verfahren zum Erhalt eines Extrakts aus Lupinenkörnerschalen, enthaltend Lupeol, **dadurch gekennzeichnet, dass** es mindestens die Abfolge von folgenden Schritten umfasst:
- Mahlen der Lupinenschalen,
- Extrahieren der Gesamtlipide, die in den gemahlenen Lupinenschalen enthalten sind, mit Hilfe eines organischen Lösungsmittels, ausgewählt aus der Gruppe bestehend aus den aliphatischen Alkanen, den aromatischen Alkanen und deren halogenierten Derivaten, und
- Reinigen der erhaltenen Lipide, um ein Lupeolreiches Extrakt zu erhalten, **dadurch gekennzeichnet, dass** die Reinigung der erhaltenen Lipide mindestens die Abfolge von folgenden Schritte umfasst:
- Verdampfen des Lösungsmittels unter Vakuum, dann Auflösen in einem aliphatischen Alkohol,
- Verseifen,
- Kristallisieren durch Abkühlen, und
- Filtrieren und/oder Waschen und/oder Zentrifugieren.

2. Verfahren zum Erhalt eines Extrakts aus Lupinenkörnerschalen, enthaltend Lupeol, **dadurch gekennzeichnet, dass** es mindestens die Abfolge von folgenden Schritten umfasst:
- Mahlen der Lupinenschalen,
- Extrahieren der Gesamtlipide, die in den gemahlenen Lupinenschalen enthalten sind, mit Hilfe eines organischen Lösungsmittels, ausgewählt aus der Gruppe bestehend aus den aliphatischen Alkanen, den aromatischen Alkanen und deren halogenierten Derivaten, und
- Reinigen der erhaltenen Lipide, um ein Lupeolreiches Extrakt zu erhalten, **dadurch gekennzeichnet, dass** die Reinigung der erhaltenen Lipide mindestens die Abfolge von folgenden Schritten umfasst:
- Verdampfen des Lösungsmittels unter Vakuum, dann auflösen in einem aliphatischen Alkohol,
- Verseifen,
- Flüssig-Flüssig-Extraktion der Unverseifbaren der Lupinenschalen mit Hilfe eines organischen Lösungsmittels, ausgewählt aus der Gruppe bestehend aus den aliphatischen Alkanen und deren halogenierten Derivaten, und
- Reinigen der Unverseifbaren durch Waschen, dann Verdampfen des Lösungmittels unter Vakuum und Trocknen.

3. Verfahren nach Anspruch 1 oder 2, **dadurch gekennzeichnet, dass** das organische Lösungmittel Hexan ist.

4. Verfahren nach Anspruch 2 oder 3, **dadurch gekennzeichnet, dass** das organische Lösungsmittel für den Schritt der Flüssig-Flüssig-Extraktion Dichlorethan ist.

5. Verfahren nach einem der Ansprüche 1 bis 4, **dadurch gekennzeichnet, dass** es außerdem einen oder mehrere Endschritt(e) zur Rekristallisierung umfasst.

6. Verfahren nach einem der vorhergehenden Ansprüche, **dadurch gekennzeichnet, dass** der erhaltene Extrakt einen Lupeolrate von mehr als 30 Gew.-%, vorzugsweise mehr als 50 Gew.-% aufweist.

## Claims

1. Method for obtaining an extract from the pods of lupin seeds containing lupeol, **characterised in that** it comprises at least the succession of the following steps:
- crushing of lupin pods,
- extraction of the total lipids contained in the crushed pods of lupin using an organic solvent chosen from the group consisting of alphatic alkanes, aromatic alkanes, and halogenated derivatives thereof, and
- purification of the lipids obtained in order to obtain a lupeol-rich extract,
**characterised in that** the purification of the lipids obtained comprises at least the succession of the following steps:
- evaporation of the solvent under vacuum, then dissolution in an aliphatic alcohol,
- saponification,
- crystallisation by cooling, and
- filtration and/or washing and/or spinning.

2. Method for obtaining an extract from the pods of lupin seeds containing lupeol, **characterised in that** it comprises at least the succession of the following steps:
- crushing of lupin pods,
- extraction of the total lipids contained in the crushed pods of lupin using an organic solvent chosen from the group consisting of alphatic alkanes, aromatic alkanes, and halogenated derivatives thereof, and
- purification of the lipids obtained in order to obtain a lupeol-rich extract,
**characterised in that** the purification of the lipids obtained comprises at least the succession of the following steps:
- evaporation of the solvent under vacuum, then dissolution in an aliphatic alcohol,
- saponification,
- liquid-liquid extraction of the unsaponifiables of pods of lupin using an organic solvent chosen from the group consisting of alphatic alkanes and halogenated derivatives thereof, and
- purification of the unsaponifiables by washing, then evaporation of the solvents under vacuum and drying.

3. Method according to claim 1 or 2, **characterised in that** the organic solvent is hexane.

4. Method according to claim 2 or 3, **characterised in that** the organic solvent for the step of liquid-liquid extraction is dichloroethane.

5. Method according to any one of claims 1 to 4, **characterised in that** it further comprises one or several final steps of recrystallisation.

6. Method according to any one of the preceding claims, **characterised in that** the extract obtained has a lupeol content greater than 30% by weight, preferably greater than 50% by weight.
